# EUROPEAN PATENT SPECIFICATION

(11) **EP 4 401 687 B1**
(45) Date of publication and mention of the grant of the patent: **05.11.2025**
(21) Application number: 21798768.4
(22) Date of filing: 15.09.2021
(51) Int. Cl.: A61F 6/08

(54) **ENDOVAGINAL DEVICE FOR THE CORRECTION OF BLADDER AND UTERINE PROLAPSE INCORPORATING A SELF-WASHING SYSTEM WITH VAGINAL DOUCHES**
ENDOVAGINALE VORRICHTUNG ZUR KORREKTUR VON BLASEN- UND GEBÄRMUTTERSENKUNG, DIE EIN SELBSTWASCHSYSTEM MIT VAGINALSPÜLUNGEN ENTHÄLT
DISPOSITIF ENDOVAGINAL POUR LA CORRECTION DU PROLAPSUS DE LA VESSIE ET DE L'UTÉRUS INCORPORANT UN SYSTÈME D'AUTO-LAVAGE AVEC DOUCHES VAGINALES

(43) Date of publication of application: 24.07.2024
(73) Proprietor: ADL FARMACEUTICI S.R.L., 20129 Milano (MI) (IT)
(72) Inventor: RAPPA, Carlo, 80126 Napoli (IT)
(74) Representative: Sarpi, Maurizio
(86) International application number: PCT/IB2021/058413
(87) International publication number: WO 2023/041956

(56) References cited:
- WO-A1-2018/213187
- WO-A2-2012/049676
- GB-A- 190 905 103
- US-B2- 7 673 631

## Description

The present invention relates to a pessary, a device for the treatment of pelvic organ prolapse (POP) in women, characterized in that it integrates a passive system aimed at providing mechanical support to the walls of the vagina with a douching system of the vagina itself by capillary douche irrigation along its walls, which can be connected to an external irrigation unit, also when the pessary is in situ, to prevent complications deriving from the prolonged presence and the repeated insertion and extraction maneuvers of the current vaginal pessaries, such as irritation, erosion, bleeding and malodorous discharge.

### Field of the invention

Pelvic organs, such as the uterus, the bladder, and the bowels, may protrude into the vagina because of weakness in the tissues which normally support them. The symptoms they cause depend on the type of prolapse. Pessaries (mechanical devices usually made of latex or silicone) may be used to attempt to restore the prolapsed organs to their normal position and thus relieve the symptoms. They are commonly used, whenever conservative treatments, such as physical therapy and surgery, have either failed or are not appropriate.

Recent studies in the sector have highlighted the fact that pelvic organ prolapse is common and is found in over 50% of multiparous women in a clinical setting (Swift 2000). In the population as a whole, it is estimated that 30% of women have signs of prolapse although the majority are asymptomatic (Samuelson 1999). These data immediately indicate how important the pathology is.

### Background art.

Various vaginal devices for POP treatment have been in use for many years. These are normally made of solid material and, to ensure proper operation, must be as wide as possible to serve as a mechanical barrier, preventing the extrusion of the pelvic organs through the introitus. This makes it difficult and painful to insert the device and then remove it for cleaning. However, this is necessary because the prolonged use of the device causes vaginal dryness and if the device is not cleaned regularly it can lead to even serious infections.

Several inflatable devices have been suggested to overcome these problems. Cf., for example, inter alia, U.S. Patent n. 2,638,093, 3,646,929, 4,019,490 and 4,920,986, and again U.K. patent n. 1,115,727.

However, all of these devices, as well as other inflation devices on the market, have two main disadvantages:
air can leak from inside the inflatable body with use, which is detrimental to the containment effectiveness of the device; and
even if the insertion and extraction operations are somehow facilitated, the problem of allowing an effective douching in situ has never been faced, making it necessary to extract the pessary for hygienic reasons, clean it thoroughly and reinsert it, reusing the device multiple times.

WO 2012/049676 A2, GB 05103 A AD 1909, and WO 2018/213187 all disclose the features of the preamble of claim 1 and describe a pessary for the correction of bladder and/or uterine prolapse support wherein the pessary comprises a multilevel body comprising a deck ergonomically shaped to adapt to the vaginal morphology, which performs the function of anti-prolapse support, a tubular element closed at its ends, which follows and is fixed to the profile of said deck and is provided with holes for the distribution of a douche on the walls of the vagina, and a tube protruding from said tubular element for introducing said douche into said tubular element from the outside, leaving said pessary in situ.

However, none of the documents of the available background art describe a pessary which is easy to insert and which can be connected to either an inflating tube or an irrigation unit.

From the above, it is apparent that there is a need for a vaginal device which offers soft and adjustable support, eliminates any difficulty during the insertion and extraction operations from the vagina, and is provided with means for spraying, once in situ, refreshing, hygienic and medical substances, to allow such an operation without needing to remove the device from the vagina each time, thus reducing the possibility of infection.

Substantially, in a first embodiment, the vaginal pessary of the present invention is made of substantially elastic material compatible with its use, consisting of a multilevel body comprising:
a deck ergonomically shaped to adapt to the vaginal morphology, which provides the function of anti-prolapse support;
a tubular element, closed at its ends, which follows and is attached to the profile of said deck, and is provided with an array of holes appropriately distanced for the distribution of a vaginal douche, and
a tube for introducing the douching liquid into such a tubular element from the outside.

For the tubular element to be integral with the deck, the latter is provided with coupling means regularly distributed along its profile.

In a second embodiment, to facilitate the introduction of the described pessary into the vagina, it is provided that the deck itself is deformable, being made up of rigid parts and inflatable parts which can be a series of parallel transversal elements or one or more axial elements, perpendicular to the preceding ones, all connected to a small inflating tube which runs along one of the side branches of the toroidal douching element and then protrudes in the same manner as the douche feeding tube.

In a third embodiment, the deck is shown by a flat inflatable air chamber surrounded by a toroidal element for the distribution of the douche, which follows the profile of said deck, there being provided a protruding tubular fitting for the inflating air of the deck and a second fitting for the introduction of the douching liquid into the toroidal distribution element.

Further features and advantages of the invention will be apparent from the following detailed description with reference to the accompanying drawings which illustrate a preferred embodiment by way of a non-limiting example of the invention.
In the figures:
figs. 1A and 1B are a perspective view from the top and the bottom of an endovaginal device;
figs. 2A and 2B are perspective views from the top and the bottom of an endovaginal device;
fig. 3 is a plan view from the top of the upper face of a vaginal device according to the invention wherein the deck consists of a single flat air chamber the distal end of which displays a succession of three protruding profiles of shockproof material to prevent possible damage to the wall of the vagina, while two tube stretches protrude from its proximal end, one for the introduction of the inflating air and the other for the egress of the douching liquid;
fig. 4 is a profile view of the vaginal device in fig. 3;

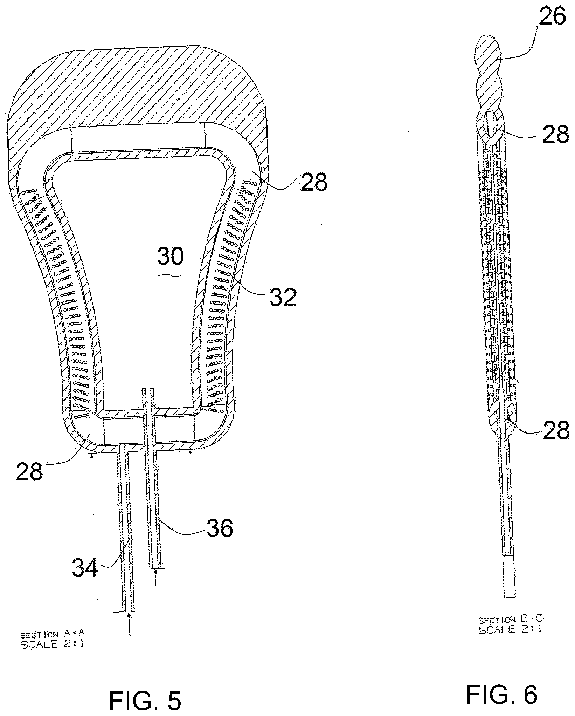

fig. 5 is a section view taken along section plane A-A in fig.4;
fig. 6 is a section view taken along section plane C-C in fig. 3;
fig. 7 is a section view taken along section plane B-B in fig. 3.

With reference to the figures, the pessary object of the invention consists of a rigid flat-shaped component or deck 2, ergonomically shaped to adapt to the vaginal morphology, which is contoured on its curved distal front end and its two mutually straight sides 4 and 6 by an extruded tube 8 of a material compatible for use, provided with a plurality of holes 10, for the distribution of vaginal douche.

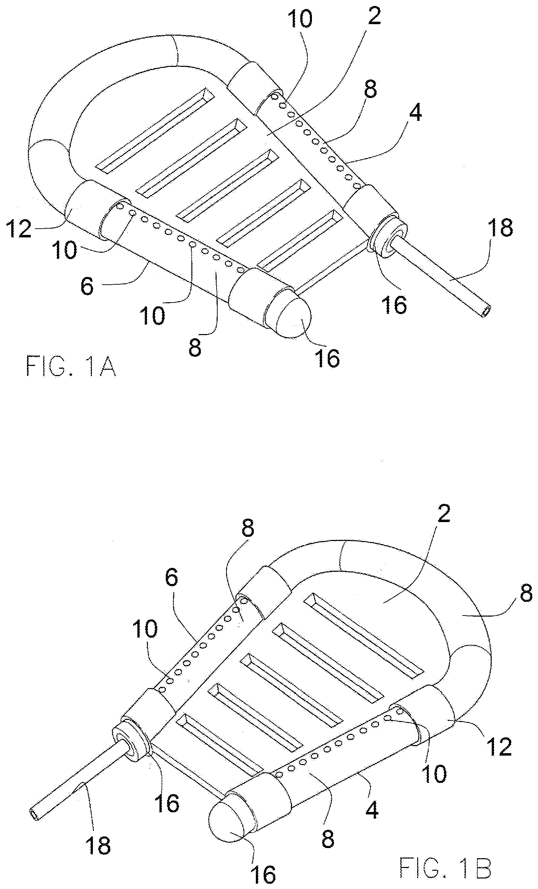

In the example shown in figs. 1A,1B, the mounting of the sprayer tube 8 on the deck 2 is achieved by virtue of the elasticity of the tube itself, which is bent to pass through sleeves 12 which protrude outside the perimeter of the deck 2, to assume its configuration. The two proximal ends of the tube are closed by caps 16, one of which is provided with a connection to a protruding feeding tube 18 which allows a vaginal douche to be introduced even when the pessary is in situ.

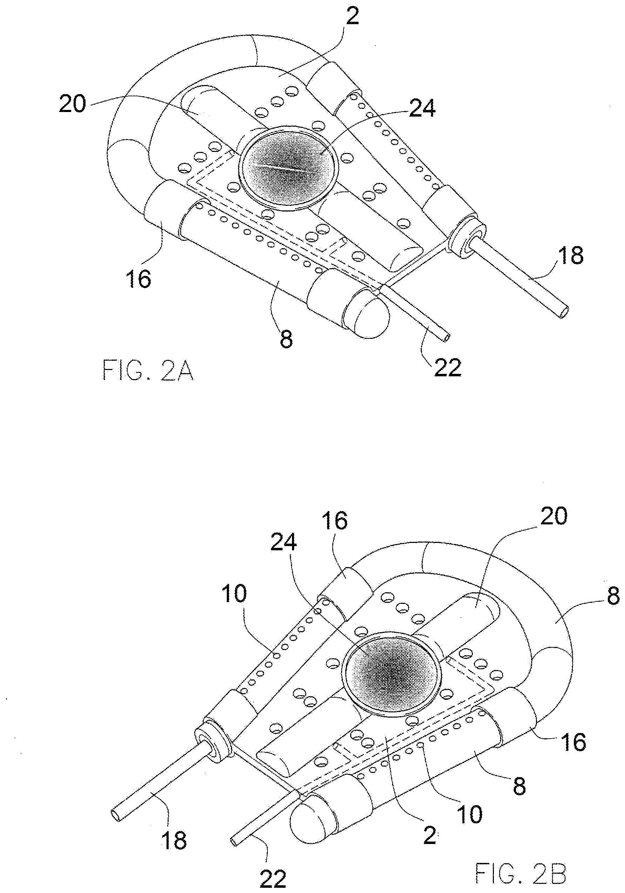

According to figs. 2A and 2B, the deck 2 is partially made of inflatable material, comprising at least one inflatable element 20 arranged axially (as in the figure), but also transversely, and even better a series of parallel transversal elements, all connected to an inflating tube 22, obviously of small diameter, which runs along the straight side opposite to the one where the douche feeding tube 18 opens. In this manner, when the inflatable element or elements either is or are deflated, it is easy to introduce the pessary into the vagina and inflate it before positioning the balloon which is responsible for correcting bladder and/or uterine prolapse. In the illustrated case, the element 20 provides the required support 24 for the anti-prolapse balloon.

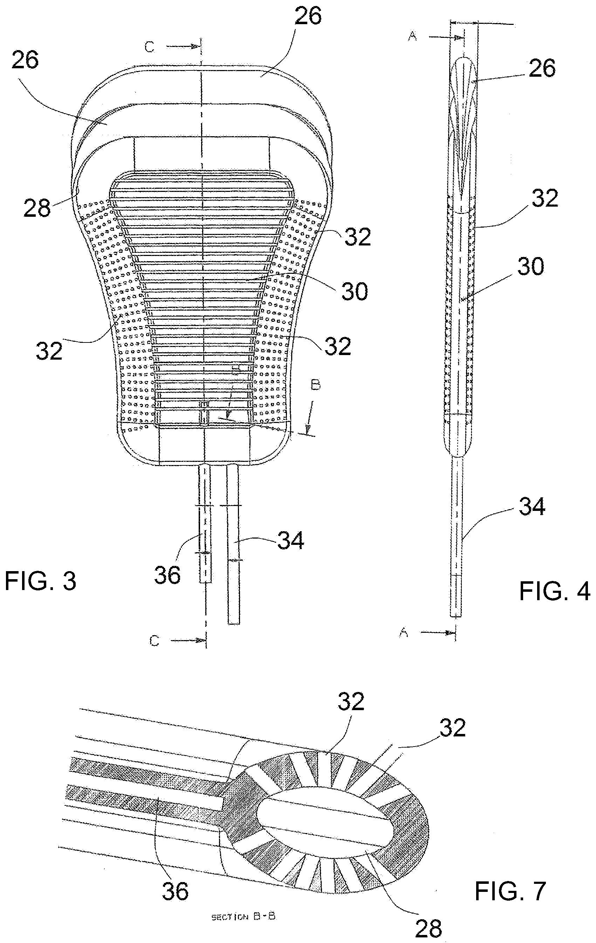

Figure 3 shows an embodiment of the present invention in which the deck comprises a single flat air chamber 30, the distal end of which comprises a succession of two full bodies 26 which embrace the tubular contour 28 of the deck, wherein a plurality of distribution channels 32 are present of the douche fed through an inlet tube 34 which runs parallel to the introduction side of the inflating air tube 36.

Such a design facilitates the insertion of the pessary into the vagina, which, once in situ, can be connected to both the inflating air tube and the douching fluid irrigation device.

An embodiment of the invention was described hereto. It is finally apparent that many variations can be made by a person skilled in the art without because of this departing from the scope of protection as defined by the appended claims.

## Claims

1. A pessary for the correction of bladder and/or uterine prolapse, consisting of a multilevel body comprising:
- a deck (2) ergonomically shaped to adapt to the vaginal morphology, which provides the function of anti-prolapse support;
- a tubular element (8), closed at its ends, which follows and is attached to the profile of said deck (2), and is provided with an array of holes (10) for the distribution on the walls of the vagina of a douche, and
- a tube (18) protruding from said tubular element (8) introduce said douche into said tubular element (8) from the outside,
**characterized in that**
the deck (2) consists of a single flat air chamber (30) with a tubular distribution profile of the douche, two stretches of tube protrude at the proximal end thereof, one for the introduction of inflating air of the deck (2) and the other for the introduction of the douching liquid into the contour (28) of the deck (2).

2. A pessary according to claim 1, wherein said tubular element (8) is a folding extruded tube and said deck (2) is equipped along its edge with gripping means to position and constrain said tubular element (8) for the distribution of the douche along its contour in order to respect its peripheral configuration.

3. A pessary according to any one of the preceding claims, wherein in order to facilitate the introduction into the vagina, the deck (2) itself is deformable, including one or more inflatable parts, connected to an inflating tube (22) protruding from said pessary configured to act as a douche feeding tube.

4. A pessary according to the preceding claim, wherein the inflatable parts of the deck (2) comprise one or more air chambers capable of forming one or more bulges in the deck (2) itself.

5. A pessary according to claim 4, wherein its distal end consists of two full bodies (26) which embrace the tubular contour (28) of the deck (2), in which there is a plurality of distribution channels for the fed douche.

## Patentansprüche

1. Pessar für die Korrektur eines Blasen- und/oder Gebärmuttervorfalls, der aus einem mehrstufigen Körper besteht, der Folgendes umfasst:
- ein Deck (2), das ergonomisch geformt ist, um sich an die Vaginalmorphologie anzupassen, das die Funktion einer Vorfallschutz-Unterstützung bereitstellt,
- ein röhrenförmiges Element (8), das an seinen Enden geschlossen ist, das dem Profil des Decks (2) folgt und an demselben befestigt ist und mit einer Gruppierung von Löchern (10) für die Verteilung einer Spülung an den Wänden der Vagina versehen ist, und
- eine Röhre (18), die von dem röhrenförmigen Element (8) aus vorspringt, um die Spülung von der Außenseite her in das röhrenförmige Element (8) einzuleiten,
**dadurch gekennzeichnet, dass**
das Deck (2) aus einer einzigen flachen Luftkammer (30) mit einem röhrenförmigen Verteilungsprofil der Spülung besteht, wobei zwei Röhrenabschnitte an dem proximalen Ende derselben vorspringen, einer für die Einleitung von Aufblasluft des Decks (2) und der andere für die Einleitung der Spülungsflüssigkeit in den Umriss (28) des Decks (2).

2. Pessar nach Anspruch 1, wobei das röhrenförmige Element (8) eine zusammenlegbare extrudierte Röhre ist und das Deck (2) entlang seines Randes mit Greifmitteln versehen ist, um das röhrenförmige Element (8) für die Verteilung der Spülung entlang seines Umrisses zu positionieren und einzugrenzen, um seine Umfangskonfiguration zu berücksichtigen.

3. Pessar nach einem der vorhergehenden Ansprüche, wobei, um die Einführung in die Vagina zu erleichtern, das Deck (2) selbst verformbar ist, wobei es einen oder mehrere aufblasbare Teile umfasst, die mit einer Aufblasröhre (22) verbunden sind, die von dem Pessar aus vorspringt, wobei sie dafür konfiguriert ist, als eine Spülungszuführungsröhre zu funktionieren.

4. Pessar nach einem der vorhergehenden Ansprüche, wobei die aufblasbaren Teile des Decks (2) eine oder mehrere Luftkammern umfassen, die dazu in der Lage sind, eine oder mehrere Aufwölbungen in dem Deck (2) selbst zu bilden.

5. Pessar nach Anspruch 4, wobei sein distales Ende aus zwei vollen Körpern (26) besteht, die den röhrenförmigen Umriss (28) des Decks (2) umschließen, in denen es eine Vielzahl von Verteilungskanälen für die zugeführte Spülung gibt.

## Revendications

1. Pessaire pour la correction d'un prolapsus vésical et/ou utérin, constitué d'un corps à plusieurs niveaux comprenant :
- une plateforme (2) de forme ergonomique pour s'adapter à la morphologie vaginale, qui assure la fonction de support anti-prolapsus ;
- un élément tubulaire (8), fermé à ses extrémités, qui suit et est fixé au profil de ladite plateforme (2), et est doté d'un réseau de trous pour la distribution d'une douche sur les parois du vagin, et
- un tube (18) faisant saillie à partir dudit élément tubulaire (8) pour introduire ladite douche dans ledit élément tubulaire (8) de l'extérieur,
**caractérisé en ce que**
la plateforme (2) est constituée d'une chambre à air plate unique (30) avec un profil de distribution tubulaire de la douche, deux étendues de tube faisant saillie à l'extrémité proximale de celle-ci, l'une pour l'introduction d'air de gonflage de la plateforme (2) et l'autre pour l'introduction du liquide de douche dans le contour (28) de la plateforme (2).

2. Pessaire selon la revendication 1, dans lequel ledit élément tubulaire (8) est un tube extrudé pliant et ladite plateforme (2) est équipée le long de son bord de moyens de préhension pour positionner et contraindre ledit élément tubulaire (8) pour la distribution de la douche le long de son contour afin de respecter sa configuration périphérique.

3. Pessaire selon l'une quelconque des revendications précédentes, dans lequel, afin de faciliter l'introduction dans le vagin, la plateforme (2) elle-même est déformable, comportant une ou plusieurs parties gonflables, reliées à un tube de gonflage (22) faisant saillie à partir dudit pessaire configuré pour agir comme un tube d'administration de douche.

4. Pessaire selon la revendication précédente, dans lequel les parties gonflables de la plateforme (2) comprennent une ou plusieurs chambres à air aptes à former une ou plusieurs bosses dans la plateforme (2) elle-même.

5. Pessaire selon la revendication 4, dans lequel son extrémité distale est constituée de deux corps pleins (26) qui enveloppent le contour tubulaire (28) de la plateforme (2), dans lequel il existe une pluralité de canaux de distribution pour la douche administrée.
